# DEMANDE DE BREVET EUROPEEN

(11) **EP 1 714 636 A1**
(43) Date de publication de la demande: **25.10.2006**
(21) Numéro de dépôt: 06290602.9
(22) Date de dépôt: 13.04.2006
(51) Int. Cl.: A61K 8/19, A61K 8/25, A61K 8/26, A61K 8/29, A61K 8/88, A61K 8/92, A61Q 1/04, A61Q 1/06

(54) **Composition cosmétique comprenant des particules de silice, des particules réfléchissantes et au moins un polymère particulier, procédé de préparation et utilisations.**

(30) Priorité: 19.04.2005 FR 0503895
(71) Demandeur: L'ORÉAL, 75008 Paris (FR)
(72) Inventeur: Ferrari, Véronique, 94700 Maisons-Alfort (FR); Khachikian, Hélène, 94140 Alfortville (FR)
(74) Mandataire: Casalonga, Axel

(57) **Abrégé**

L'invention concerne une composition cosmétique de maquillage et/ou de soin de la peau contenant une phase grasse notamment gélifiée ou structurée comprenant des particules de silice et des particules réfléchissantes, telle que ladite phase grasse comprend au moins un polymère de masse moléculaire moyenne en poids inférieure à 100 000 et mieux inférieure à 50 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

L'invention concerne encore un procédé de préparation de cette composition cosmétique, un gloss comprenant cette composition cosmétique ainsi que l'utilisation de cette composition cosmétique pour l'obtention d'un dépôt brillant.

## Description

La présente invention se rapporte au domaine du maquillage et/ou du soin de la peau et plus particulièrement au domaine des compositions de maquillage et/ou de soin de la peau comprenant une phase grasse contenant des particules de silice.

La présente demande concerne des compositions dans lesquelles les particules de silice sont utilisées notamment en tant que système gélifiant principal, cependant la présence d'un gélifiant supplémentaire dans ces compositions n'est pas exclue.

Les compositions de maquillage et/ou de soin de la peau comprenant une phase grasse gélifiée ou structurée telles que les fonds de teint, les fards à joues, les fards à paupières, les rouges à lèvres sont couramment utilisées pour remodeler le visage, en particulier pour mettre en valeur les pommettes ou pour rendre les lèvres pulpeuses.

Or ces compositions de maquillage et/ou de soin de la peau présentent généralement une sensibilité au cisaillement notamment lors de leur fabrication, et, lorsque ces compositions comprennent des particules en suspension, la sensibilité au cisaillement se traduit par une tendance de ces particules à sédimenter lors de leur stockage.

En particulier, ces compositions comprenant une phase grasse gélifiée ou structurée peuvent être utilisées dans des compositions de maquillage des lèvres de type « gloss » car la gélification de la phase conduit à des systèmes opalescents. Cette transparence revêt un intérêt supplémentaire si les indices de réfraction des huiles de la phase grasse ont été choisis de façon à permettre un dépôt brillant sur les lèvres.

Il existe par conséquent un besoin de disposer de compositions de maquillage et/ou de soin de la peau comprenant une phase grasse notamment gélifiée ou structurée au moyen de particules de silice, ces compositions présentant une homogénéité et une stabilité améliorée. En particulier, lorsque ces compositions comprennent des particules en suspension, il existe un besoin d'obtenir une amélioration de la stabilité de la suspension des particules, c'est-à-dire un besoin d'obtenir une composition dans laquelle les particules n'ont pas tendance à sédimenter.

De manière surprenante et avantageuse, la demanderesse a découvert que le problème de la stabilité de ces compositions peut être résolu en utilisant un polymère particulier qui est un polymère de masse moléculaire moyenne en poids inférieure à 100 000 et mieux inférieure à 50 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

Un premier objet de la présente demande est une composition cosmétique de maquillage et/ou de soin de la peau contenant une phase grasse comprenant des particules de silice et des particules réfléchissantes en suspension ainsi qu'au moins un polymère de masse moléculaire moyenne en poids inférieure à 100 000 et mieux inférieure à 50 000, ce polymère comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

Un deuxième objet de la présente demande consiste en un procédé de préparation de cette composition cosmétique tel que l'on mélange des particules de silice, des particules réfléchissantes et au moins un polymère de masse moléculaire moyenne en poids inférieure à 100 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

Un avantage de la présente demande est qu'il est possible d'obtenir des compositions transparentes. Cette propriété prend tout son intérêt lorsque ladite composition est utilisée pour fabriquer un « gloss » car dans ce cas, les propriétés de brillance des matériaux utilisés tels que les huiles de la phase grasse ou encore les particules réfléchissantes se trouvent conservées voire améliorées.

En conséquence, un troisième objet de la présente demande consiste en un gloss comprenant cette composition cosmétique.

Un quatrième objet de la présente demande consiste en l'utilisation de cette composition cosmétique pour l'obtention d'un dépôt brillant.

D'autres caractéristiques, aspects, objets et avantages de la présente invention apparaîtront encore plus clairement à la lecture de la description et des exemples qui suivent.

De préférence, la phase grasse de la composition selon la présente demande est gélifiée ou structurée.

Par « phase grasse gélifiée» au sens de la présente demande on entend que ladite phase est sous forme d'un gel, c'est-à-dire un réseau tridimensionnel de molécules qui retient dans ses mailles une quantité importante de solvant.

La formation d'un tel réseau constitue sa gélification.

Par « phase grasse structurée» au sens de la présente demande on entend que ladite phase est un gel rigide, présenté en coupelle ou en stick.

La phase gélifiée ou structurée utilisée dans la composition selon la présente demande présente une viscosité dynamique à température ambiante comprise entre 30 et 60 Pa.s, de préférence entre 40 et 60 Pa.s.

La viscosité dynamique de la composition est mesurée avec un viscosimètre de type METTLER RM 180. L'appareil METTLER RM 180 (Rhéomat) peut être équipé de différents mobiles en fonction de l'ordre de grandeur de la viscosité que l'on veut mesurer. Pour une viscosité comprise entre 8 et 122 Pa.s, on équipe l'appareil d'un mobile 5. La vitesse de rotation du mobile est de 200 tours/min.

Selon une variante avantageuse, les compositions selon la présente demande sont transparentes, cette transparence est évaluée visuellement sur la base d'une épaisseur de couche de 10 µm. Cette épaisseur correspond sensiblement à l'épaisseur d'un dépôt de maquillage obtenu par exemple avec un fond de teint ou un rouge à lèvres de préférence de type « gloss ».

On désigne par « gloss » un produit destiné à être appliqué sur les lèvres et conditionné par exemple dans un récipient pourvu d'un applicateur, cet applicateur comportant un organe de préhension qui sert également de capuchon de fermeture du récipient.

Les compositions selon la présente invention comprennent un milieu physiologiquement acceptable c'est-à-dire un milieu non toxique et susceptible d'être appliqué sur la peau, les lèvres ou les phanères d'un être humain.

De façon préférée, la composition cosmétique comprend une phase grasse d'indice de réfraction compris entre 1,47 et 1,51, ce qui peut permettre d'obtenir une brillance relativement élevée. Ladite phase grasse de la composition selon l'invention comprend des particules de silice; de préférence la phase grasse est gélifiée ou structurée au moyen de particules de silice.

Les particules de silice peuvent être notamment choisies parmi la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6^{ème} édition, 1995^{).} Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références Aerosil R972®, et Aerosil R974® par la société DEGUSSA, CAB-O-SIL TS-610® et CAB-O-SIL TS-720® par la société CABOT

La silice pyrogénée hydrophobe présente en particulier une taille de particules pouvant être nanométrique à micrométrique, par exemple allant d'environ de 5 à 200 nm.

La quantité de particules de silice peut représenter de 0,1 à 12%, de préférence de 0,5 à 10, de préférence encore de 6 à 8% en poids par rapport au poids total de la composition.

Les particules réfléchissantes utilisées doivent être compatibles avec une utilisation en cosmétique et doivent pouvoir subsister dans le milieu physiologiquement acceptable, et notamment ne pas se dissoudre, ou en tout cas ne pas se dissoudre entièrement, dans celui-ci.

De préférence elles sont choisies dans le groupe constitué par : les particules à substrat naturel ou synthétique, enrobé au moins partiellement d'au moins une couche d'au moins un métal, les particules à substrat synthétique enrobé au moins partiellement d'au moins une couche d'au moins un composé métallique et notamment d'un oxyde métallique, les particules formées d'un empilement d'au moins deux couches à indices de réfraction différents, notamment deux couches de polymères, et les particules d'oxydes métalliques.

Les particules réfléchissantes peuvent être présentes dans la composition, de préférence en étant dispersées de manière homogène, par exemple à une teneur allant de 0,1 % à 20 % par rapport au poids total de la composition, de préférence de 1 % à 15 % en poids, et mieux de 1 % à 10 % en poids, par exemple environ 2 % notamment pour une composition destinée à être appliquée sur les lèvres.

La teneur en particules réfléchissantes pourra dépendre, entre autres, de la nature du support destiné à recevoir la composition cosmétique, ainsi que de la nature du milieu physiologiquement acceptable et de la nature et de la taille des particules réfléchissantes. La teneur en particules réfléchissantes sera choisie de préférence de manière à ce que les points de surbrillance soient répartis de manière discrète sur la surface à maquiller/soigner. Les particules réfléchissantes peuvent être dans une quantité suffisante pour que l'on puisse observer simultanément, lorsque la composition cosmétique est appliquée sur un support tel que les lèvres par exemple, une pluralité de points de surbrillance, par exemple plus d'une dizaine, voire plus d'une cinquantaine, ou plus encore, par exemple plus d'une centaine ou plusieurs centaines.

Leur taille est compatible avec la manifestation d'une réflexion spéculaire de la lumière visible (400-700 nm), d'intensité suffisante, compte tenu de la brillance moyenne de la composition, pour créer un point de surbrillance. Cette taille est susceptible de varier selon la nature chimique des particules, leur forme et leur pouvoir de réflexion spéculaire de la lumière visible.

Parmi les particules réfléchissantes utilisables dans l'invention, certaines peuvent présenter un écart relatif Δ, défini par la formule Δ= [L*_{SCI} - L*_{SCE]} / L*_{SCE}, supérieur ou égal à 0,25. Par comparaison, certaines nacres qui ne conviennent pas en tant que particules réfléchissantes présentent un coefficient Δ inférieur à 0,25. Dans la formule ci-dessus, L*_{SCI} désigne la clarté L* mesurée à l'aide d'un spectrocolorimètre de marque MINOLTA et de référence CM-2002, dans un mode dit « composante spéculaire incluse », et L*_{SCE}, la clarté L* mesurée à l'aide du même appareil, dans un mode dit « composante spéculaire exclue ». Pour effectuer les mesures, on réalise une dispersion à 5 % en poids des particules à tester dans un vernis à ongles transparent de composition classique (essentiellement de la nitrocellulose, une résine et un plastifiant) et l'on étale à l'état fluide une couche de 300 µm d'épaisseur de la composition ainsi formée sur le fond noir d'une carte de contraste.

On utilise la fonction SCI/SCE du spectrocolorimètre avec la géométrie d/8 pour mesurer L*_{SCI} et L*_{SCE}.

A titre d'exemple, on a mesuré pour des particules réfléchissantes de marque REFLECKS®, commercialisées par la société ENGELHARD, comportant un substrat de verre enrobé d'oxyde de fer brun, un écart relatif Δ supérieur à 0,7 alors que pour des nacres FLAMENCO® commercialisées par la même société on a mesuré un écart relatif inférieur à 0,2.

Les particules réfléchissantes présenteront de préférence une dimension d'au moins 10 µm, par exemple comprise entre environ 20 µm et environ 80 µm.

Par « dimension », on désigne la dimension donnée par la distribution granulométrique statistique à la moitié de la population, dite D50. La taille des particules réfléchissantes pourra dépendre de leur état de surface. Plus celui-ci est réfléchissant, plus la dimension pourra *a priori* être faible, et inversement.

Dans un souci d'esthétique, il est préférable que, sauf lorsqu'elles brillent pour créer des points de surbrillance, les particules réfléchissantes ne soient pas perceptibles du tout ou pas aisément perceptibles à l'oeil nu à la surface de la composition appliquée sur son support. Il est également souhaitable que les particules réfléchissantes ne soient pas de dimensions telles qu'elles créent une sensation d'inconfort sur le support. L'utilisation de particules de taille inférieure ou égale à 250 µm, et mieux inférieure ou égale à 150 µm, par exemple inférieure à 100 µm, est ainsi privilégiée. La taille des particules pourra également dépendre de la nature du support sur lequel la composition est destinée à être appliquée ; certaines parties du corps ou du visage pourront par exemple plus facilement que d'autres tolérer des dimensions plus grandes sans générer d'inconfort.

Les particules réfléchissantes peuvent présenter des formes variées. Ces particules peuvent être notamment en forme de plaquettes ou globulaires, en particulier sphériques.

Par l'expression « en forme de plaquette », on désigne des particules dont le rapport de la plus grande dimension à l'épaisseur est supérieur ou égal à 5, voire 10 ou mieux 20. L'épaisseur des particules en forme de plaquettes est par exemple comprise entre environ 0,5 µm et environ 5 µm.

Les particules présentant une surface extérieure sensiblement plane conviennent tout particulièrement, car elles peuvent plus facilement donner naissance, si leur taille, leur structure et leur état de surface le permet, à une réflexion spéculaire intense. On parle d'effet miroir.

Pour de telles particules notamment, c'est essentiellement la lumière renvoyée par réflexion dans une direction faisant, avec la normale à la surface réfléchissante, le même angle que celui que fait la lumière incidente avec cette normale, qui permet à ces particules d'apparaître comme des points de surbrillance, et non la lumière diffusée dans les autres directions.

Il peut être souhaitable que les particules réfléchissantes soient non diffusantes et non mates.

Il peut être souhaitable également que les particules réfléchissantes n'altèrent pas de manière sensible la coloration de la composition cosmétique.

A cet égard, les particules réfléchissantes qui permettent une réflexion métallique de la lumière incidente conviennent tout particulièrement. C'est le cas notamment lorsque les particules réfléchissantes permettent, quelque soit leur forme, une réflexion sur une couche d'un métal, par exemple de l'argent. De telles particules s'avèrent relativement neutres vis-à-vis de la couleur de la composition.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm (le visible), et mieux d'au moins 80 %, voire 90 % ou encore 95 %.

La lumière réfléchie par les particules réfléchissantes peut être non iridescente, notamment dans le cas d'un reflet métallique.

Les particules réfléchissantes, quelque soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, par exemple des oxydes de titane ou de fer obtenus par synthèse de manière à présenter une surface sensiblement plane ayant un état de surface, par exemple non mat et non diffusant, permettant une réflexion spéculaire de la lumière suffisante pour obtenir des points de surbrillance au sein de la composition cosmétique.

Lorsque les particules réfléchissantes présentent une structure multicouche, celles-ci peuvent par exemple comporter un substrat naturel ou synthétique, notamment un substrat synthétique au moins partiellement enrobé par au moins une couche d'au moins un matériau réfléchissant tel qu'un métal.

Quelque soit la forme des particules réfléchissantes, le substrat peut, lorsqu'il est synthétique, être réalisé avec une forme favorisant la formation d'une surface réfléchissante après revêtement, notamment après dépôt d'une couche de matériau réfléchissant. Le substrat peut, par exemple, présenter une surface plane et la couche de matériau réfléchissant une épaisseur sensiblement uniforme.

Le substrat peut être monomatière ou multimatériaux, plein ou creux. Le substrat peut être organique ou inorganique. Le substrat peut être naturel mais de préférence on utilise un substrat synthétique, pour la raison indiquée ci-dessus.

Le substrat peut être choisi par les verres, les céramiques, le graphite, les oxydes métalliques, les alumines, les silices, les silicates, notamment les aluminosilicates et les borosilicates et le mica synthétique, cette liste n'étant pas limitative.

Le matériau réfléchissant peut comporter une couche de métal ou d'un composé métallique.

La couche de métal ou de composé métallique peut enrober ou non en totalité le substrat et la couche de métal peut être au moins partiellement recouverte par une couche d'un autre matériau, par exemple un matériau transparent. Il peut être préférable que la couche de métal ou de composé métallique enrobe en totalité, directement ou indirectement, c'est-à-dire avec interposition d'au moins une couche intermédiaire, métallique ou non, le substrat.

Le métal peut être choisi par exemple parmi Ag, Au, Cu, Al, Ni, Sn, Mg, Cr, Mo, Ti, Pt, Va, Rb, W, Zn, Ge, Te, Se et leurs alliages. Ag, Au, Al, Zn, Ni, Mo, Cr, Cu et leurs alliages (par exemple les bronzes et les laitons) sont des métaux préférés.

Dans le cas notamment de particules à substrat enrobé d'argent ou d'or, la couche métallique peut être présente à une teneur représentant par exemple de 0,1 à 50 % du poids total des particules, voire entre 1 et 20 %.

Des particules de verre recouvertes d'une couche métallique peuvent avoir une dimension allant par exemple de 10 µm à 300 µm, et mieux de 25 µm à 150 µm. Dans le cas où ces particules sont en forme de plaquettes, l'épaisseur peut être comprise par exemple entre environ 0,1 µm et environ 25 µm, de préférence d'environ 0,5 µm à environ 10 µm et mieux d'environ 0,5 µm à environ 5 µm. Dans le cas où ces particules se trouvent sous forme de sphères, elles peuvent avoir une dimension allant par exemple d'environ 10 à 100 µm.

Des particules de verre recouvertes d'une couche métallique sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer encore les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelque soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, soit d'oxyde de titane, d'oxyde d'étain ou d'un de leurs mélanges comme celles vendues sous la marque REFLECKS® par la société ENGELHARD.

Conviennent également à l'invention, les pigments de la gamme METASHINE tels que les références MC 1120, 1080, 1040, 1020, ME 2040 et MC 2080 commercialisés par la société NIPPON SHEET GLASS CO. LTD. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.

On peut encore citer les particules de dimension comprise entre 80 et 100 µm, comportant un substrat de mica synthétique (fluorophlogopite) revêtu de dioxyde de titane représentant 12% du poids total de la particule, vendues sous la dénomination PROMINENCE par la société NIHON KOKEN.

Les particules réfléchissantes peuvent encore être choisies parmi les particules formées d'un empilement d'au moins deux couches à indices de réfraction différents.

Ces couches peuvent être de nature polymérique ou métallique et notamment inclure au moins une couche polymérique.

Ainsi, les particules réfléchissantes peuvent être des particules dérivant d'un film polymérique multicouche.

De telles particules sont notamment décrites dans WO 99/36477, US 6 299 979 et US 6 387 498.

A titre illustratif des matériaux pouvant constituer les différentes couches de la structure multicouche, on peut citer, cette liste n'étant pas limitative: le naphtalate de polyéthylène (PEN) et ses isomères par exemple 2,6-, 1,4-, 1,5-, 2,7- et 2,3-PEN, les téréphtalates de polyalkylène, des polyimides, des polyétherimides, des polystyrènes atactiques, des polycarbonates, des polyméthacrylates et des polyacrylates d'alkyle, du polystyrène syndiotactique (sPS), des polyalpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphtalates de polyalkylène et des copolymères de styrène. En outre, chaque couche individuelle peut inclure des mélanges de deux ou plusieurs polymères ou copolymères précédents.

Le choix des matériaux destinés à constituer les différentes couches de la structure multicouche est bien entendu effectué de manière à conférer l'aspect réfléchissant souhaité aux particules ainsi formées.

Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.

La brillance des particules réfléchissantes peut être encore due, en variante où additionnellement, à la réflexion de la lumière sur une couche d'un matériau de la particule présentant un indice de réfraction suffisant grand par rapport à celui du milieu d'où provient la lumière incidente.

La composition cosmétique selon l'invention peut bien entendu comporter des particules réfléchissantes de différentes natures sans que l'on sorte du cadre de la présente invention.

La phase grasse des compositions selon la présente invention comprend au moins un polymère de masse moléculaire moyenne en poids inférieure à 100 000 et mieux inférieure à 50 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Selon l'invention, ces chaînes peuvent être liées directement au squelette polymérique ou via une fonction ester ou un groupement perfluoré.

Par "polymère", on entend au sens de l'invention un composé ayant au moins 2 motifs de répétition, et de préférence au moins 3 motifs de répétition.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes avantageusement non pendants et se trouvant dans le squelette polymérique. Ces hétéroatomes sont choisis parmi les atomes d'azote, de soufre, de phosphore et leurs associations, associés éventuellement à un ou plusieurs atomes d'oxygène. De préférence, les motifs comportent au moins un atome d'azote en particulier non pendant. Ces motifs comportent, en outre, avantageusement, un groupe carbonyle.

Les motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique.

Ce polymère peut comprendre entre les motifs hydrocarbonés des motifs siliconés ou des motifs oxyalkylénés.

En outre, ce polymère de la composition de l'invention comprend avantageusement de 40 à 98 % de chaînes grasses par rapport au nombre total des motifs à hétéroatome et des chaînes grasses et mieux de 50 à 95 %. La nature et la proportion des motifs à hétéroatome est fonction de la nature de la phase grasse et est en particulier similaire à la nature polaire de la phase grasse. Ainsi, plus les motifs à hétéroatome sont polaires et en proportion élevée dans ce polymère, ce qui correspond à la présence de plusieurs hétéroatomes, plus ce polymère a de l'affinité avec les huiles polaires. En revanche, plus les motifs à hétéroatome sont peu polaires voire apolaires ou en proportion faible, plus ce polymère a de l'affinité avec les huiles apolaires.

Ce polymère est avantageusement un polyamide. Aussi, l'invention a également pour objet une composition contenant, dans un milieu cosmétiquement acceptable, au moins un polymère de polyamide de masse moléculaire moyenne en poids inférieure à 100 000, comportant a) un squelette polymérique, ayant des motifs répétitifs amide, et b) éventuellement au moins une chaîne grasse pendante et/ou au moins une chaîne terminale éventuellement fonctionnalisées, ayant de 8 à 120 atomes de carbone et étant liées à ces motifs amide.

De préférence, les chaînes grasses pendantes sont liées à l'un au moins des atomes d'azote des motifs amide de ce polymère.

En particulier, les chaînes grasses de ce polyamide représentent de 40 à 98 % du nombre total des motifs amide et des chaînes grasses, et mieux de 50 à 95 %.

Avantageusement, ce polymère, et en particulier ce polyamide, de la composition selon l'invention présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000, et mieux de 2000 à 10 000.

Ce polymère, et en particulier ce polyamide, est non soluble dans l'eau, notamment à 25 °C. En particulier, il ne comporte pas de groupes ioniques.

En tant que polymères préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison en particulier ester. De préférence, ces polymères comportent une chaîne grasse à chaque extrémité du squelette polymérique et en particulier du squelette polyamide. Comme autres groupes de liaison on peut citer les groupes éther, amine, urée, uréthane, thioester, thiourée, thiouréthane.

Ces polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone). Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I) suivante : 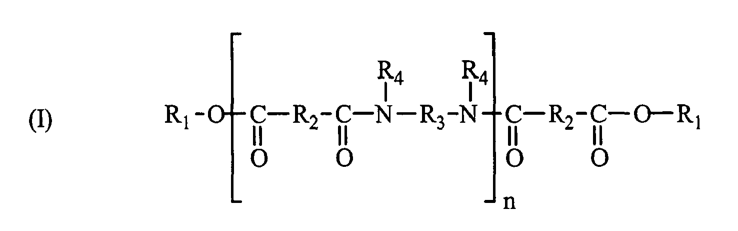 dans laquelle n désigne un nombre entier de motifs amide tel que le nombre de groupes ester représente de 10 % à 50 % du nombre total des groupes ester et amide ; R₁ est à chaque occurrence indépendamment un groupe alkyle ou alcényle ayant au moins 4 atomes de carbone et notamment de 4 à 24 atomes de carbone ; R₂ représente à chaque occurrence indépendamment un groupe hydrocarboné en C₄ à C₄₂ à condition que 50 % des groupes R₂ représentent un groupe hydrocarboné en C₃₀ à C₄₂ ; R₃ représente à chaque occurrence indépendamment un groupe organique pourvu d'au moins 2 atomes de carbone, d'atomes d'hydrogène et optionnellement d'un ou plusieurs atomes d'oxygène ou d'azote ; et R₄ représente à chaque occurrence indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure hétérocyclique définie par R₄-N-R₃, avec au moins 50 % des R₄ représentant un atome d'hydrogène.

Dans le cas particulier de la formule (I), les chaînes grasses terminales éventuellement fonctionnalisées au sens de l'invention sont des chaînes terminales liées au dernier hétéroatome, ici l'azote, du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, R₁ est un groupe alkyle en C₁₂ à C₂₂ et de préférence en C₁₆ à C₂₂. Avantageusement, R₂ peut être un groupe hydrocarboné (alkylène) en C₁₀ à C₄₂. De préférence, 50 % au moins et mieux au moins 75 % des R₂ sont des groupes ayant de 30 à 42 atomes de carbone. Les autres R₂ sont des groupes hydrogénés en C₄ à C₁₉ et même en C₄ à C₁₂. De préférence, R₃ représente un groupe hydrocarboné en C₂ à C₃₆ ou un groupe polyoxyalkyléné et R₄ représente un atome d'hydrogène. De préférence, R₃ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

A titre d'exemple de polymères utilisables dans les compositions selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94 °C. Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme polymère utilisable dans les compositions selon l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Le polymère présent dans la composition selon l'invention a avantageusement une température de ramollissement supérieure à 65°C et pouvant aller jusqu'à 190 °C. De préférence, il présente une température de ramollissement allant de 70 à 130 °C et mieux de 80 à 105 °C. Le premier polymère est en particulier un polymère non cireux.

Le(s) polymère(s) utilisable(s) selon l'invention présente(nt) du fait de leur(s) chaîne(s) grasse(s) une bonne solubilité dans les huiles et donc conduise(nt) à des compositions macroscopiquement homogènes même avec un taux élevé (au moins 25%) de polymère.

Ce polymère peut être présent dans la composition selon l'invention en une teneur allant de 0,01 % à 10 % en poids, par rapport au poids total de la composition, de préférence allant de 0,05 % à 5 % en poids, et mieux allant de 0,1 % à 3 % en poids.

Selon un mode de réalisation, le ratio massique entre le polymère et les particules de silice va de 1 :1000 à 1 :1, de préférence de 1 :100 à 1 :10, et de manière encore plus préférée de 5 :1000 à 5 :100.

De préférence, la phase grasse comprend une phase grasse liquide à température ambiante telle que celles classiquement utilisées en cosmétique. Cette phase grasse peut contenir des huiles polaires et/ou des huiles apolaires.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, ou de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule RₐCOOR_{b} dans laquelle Rₐ représente le reste d'un acide gras linéaire ou ramifié comportant de 1 à 40 atomes de carbone et R_{b} représente une chaîne hydrocarbonée, notamment ramifiée, contenant de 1 à 40 atomes de carbone à condition que Rₐ + R_{b} soit ≥ 10, comme par exemple l'huile de Purcellin (octanoate de cétostéaryle), l'isononanoate d'isononyle, le benzoate d'alcool en C₁₂ à C₁₅, le myristate d'isopropyle, le palmitate de 2-éthylhexyle, l'isostéarate d'isostéaryle, les octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₆ comme l'alcool oléique ;
- les acides gras en C₈ à C₂₆ comme l'acide oléique, linolénique et linoléique ; et
- leurs mélanges.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle ou alcoxy, latéraux et/ou en bout de chaîne, groupements ayant chacun de 2 à 24 atomes de carbone ; les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyltriméthylsiloxysilicates ; les hydrocarbures linéaires ou ramifiés d'origine synthétique ou minérale, volatils ou non, tels que les huiles de paraffine volatiles (isoparaffines comme l'isododécane) ou non volatiles, et leurs dérivés, la vaseline, la lanoline liquide, les polydécènes, le polyisobutène hydrogéné tel que l'huile de Parléam, le squalane ou l'huile d'arara ; et leurs mélanges.

De préférence, les huiles sont des huiles apolaires et plus spécialement une huile ou un mélange d'huiles du type hydrocarboné d'origine minérale ou synthétique, choisies en particulier parmi les alcanes comme l'huile de Parléam, les isoparaffines comme l'isododécane, le squalane et leurs mélanges. Avantageusement, ces huiles sont associées à une ou plusieurs huiles de silicones phénylées.

De préférence, la phase grasse liquide contient au moins une huile non volatile choisie en particulier parmi les huiles hydrocarbonées d'origine minérale, végétale ou synthétique, les esters ou éthers de synthèse, les huiles de silicone et leurs mélanges.

La phase grasse liquide totale représente, en pratique, de 5 à 99,95 %, de préférence de 10 à 80 %, et mieux de 20 à 75 % du poids total de la composition.

Les compositions selon la présente demande peuvent également comprendre des pigments, des nacres et/ou des laques.

Par pigments, on entend des particules blanches ou colorées, minérales ou organiques, enrobées ou non. On peut citer par exemple les dioxydes de titane, de zirconium ou de cérium, les oxydes de zinc, de fer ou de chrome, le bleu ferrique, l'hydrate de chrome, le noir de carbone, les outremers (polysulfures d'aluminosilicates), le violet de manganèse, le pyrophosphate de manganèse et certaines poudres métalliques telles que les poudres d'argent ou d'aluminium, et leurs mélanges.

Par nacres, on entend des pigments nacrés blancs tels que le mica recouvert d'oxyde de titane ou d'oxychlorure de bismuth et des pigments nacrés colorés tels que le micatitane recouvert avec des oxydes de fer, du bleu ferrique ou de l'oxyde de chrome, ou avec un pigment organique type précipité.

Les laques utilisables dans les compositions de la présente invention sont par exemple les laques à base de carmin de cochenille ou à base de sels de calcium, de baryum, d'aluminium, de strontium ou de zirconium, de colorants acides, et leurs mélanges.

Les compositions cosmétiques selon la présente invention contiennent généralement de 0,05 % à 20 % en poids et de préférence de 0,1 à 15 % en poids d'agent(s) choisis parmi les pigments, les laques et les nacres par rapport au poids total de la composition cosmétique.

Dans un mode de réalisation préféré de la présente invention, la composition cosmétique comprend également un ou plusieurs actifs cosmétiques. Comme actifs cosmétiques utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, ou leurs mélanges.

La présente invention est illustrée à l'aide des exemples ci-après. Ces exemples ne constituent en aucun cas une limitation de la présente invention.

### EXEMPLES

Le mode opératoire utilisé est le suivant :
Les pigments sont broyés à l'aide d'un broyeur tricylindre dans les corps gras liquides.
Lorsque les pigments sont broyés, on ajoute le polybutène puis on agite au moyen d'un agitateur de type Rayneri en portant le mélange à 100-105°C.
Une fois le mélange homogène, on ajoute des nacres et le parfum puis la silice est introduite progressivement en maintenant à 100-105°C et sous agitation. La mise en suspension de la silice doit durer environ 20 minutes pour 300 g de produit. Le produit obtenu est alors coulé dans des bouillottes soit directement à chaud, soit après refroidissement.
Le polymère Uniclear® 100 est ensuite introduit.

### Exemple 1

On prépare un produit de maquillage pour les lèvres dont la composition est la suivante :

| Polybutène | qsp |
|---|---|
| di-isostéarylmalate | 9 |
| pentaérythrityl isostéarate | 14 |
| tridécyltrimellitate | 11 |
| triglycéride d'acide en C₁₈₋₃₆ | 20 |
| bis diglycérylpolyacyladipate 2 | 18 |
| Uniclear® 100 | 0,3 |
| Silice | 8 |
| Conservateur | 0,51 |
| Pigment, nacres | 6,95 |
| Metashine | 8 |
| parfum | 0,3 |

Uniclear® 100 : condensat d'un diacide en C₃₆ hydrogéné et d'éthylènediamine, estérifié par l'alcool stéarylique (masse molaire moyenne en poids environ 4000) commercialisé par la société ARIZONA CHEMICAL.

Après conservation de ce produit pendant 3 mois à température ambiante, aucune sédimentation des particules (Pigment, nacres, metashine) n'est observée.

### Exemple 2

On prépare un produit de maquillage pour les lèvres dont la composition est la suivante :

| | |
|---|---|
| Polybutène | qsp |
| di-isostéarylmalate | 9 |
| pentaérythrityl isostéarate | 14 |
| tridécyltrimellitate | 11 |
| triglycéride d'acide en C₁₈₋₃₆ | 20 |
| bis diglycérylpolyacyladipate 2 | 18 |
| Uniclear® 100 | 0,5 |
| Silice | 8 |
| Conservateur | 0,51 |
| Pigment, nacres | 6,95 |
| Metashine | 8 |
| parfum | 0,3 |

Après conservation de ce produit pendant 3 mois à température ambiante, aucune sédimentation des particules (Pigment, nacres, metashine) n'est observée.

## Revendications

1. Composition cosmétique de maquillage et/ou de soin de la peau contenant une phase grasse comprenant des particules de silice et des particules réfléchissantes en suspension, **caractérisée en ce que** la phase grasse comprend au moins un polymère de masse moléculaire moyenne en poids inférieure à 100 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

2. Composition cosmétique selon la revendication 1 telle que le polymère est un polyamide de masse moléculaire moyenne en poids inférieure à 100 000, comportant a) un squelette polymérique ayant des motifs de répétition hydrocarbonés qui sont des amides, et b) éventuellement au moins une chaîne grasse pendante et/ou au moins une chaîne grasse terminale éventuellement fonctionnalisées, ayant de 8 à 120 atomes de carbone et étant liées à ces motifs hydrocarbonés qui sont des amides.

3. Composition selon la revendication précédente, **caractérisée par le fait que** les chaînes grasses représentent de 40 à 98 % du nombre total des motifs amide et des chaînes grasses.

4. Composition selon la revendication 2 ou 3, **caractérisée par le fait que** les chaînes grasses représentent de 50 à 95 % du nombre total des motifs amide et des chaînes grasses.

5. Composition selon l'une des revendications 2 à 4, **caractérisée par le fait que** les chaînes grasses pendantes sont liées directement à l'un au moins des atomes d'azote des motifs amide.

6. Composition selon l'une des revendications précédentes, **caractérisée par le fait que** la masse molaire moyenne du premier polymère va de 1000 à 100 000, de préférence de 1000 à 50 000, et mieux de 1 000 à 30 000.

7. Composition selon l'une des revendications précédentes, **caractérisée par le fait que** la masse molaire moyenne en poids du premier polymère filmogène va de 2 000 à 20 000, et de préférence de 2 000 à 10 000.

8. Composition selon l'une des revendications précédentes, **caractérisée par le fait que** la ou les chaînes grasses terminales sont liées au squelette par des groupes de liaison.

9. Composition selon la revendication 8, **caractérisée par le fait que** les groupes de liaison sont des groupes ester.

10. Composition selon l'une des revendications précédentes, **caractérisée par le fait que** la ou les chaînes grasses ont de 12 à 68 atomes de carbone.

11. Composition cosmétique selon l'une des revendications précédentes, **caractérisée par le fait que** les polyamides correspondent à la formule (I) : 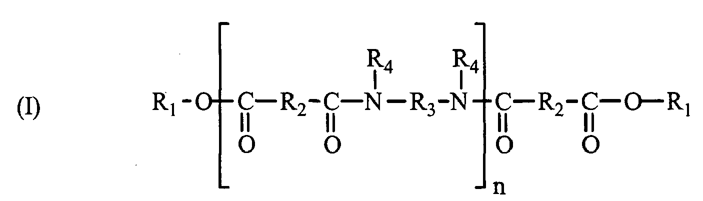 dans laquelle n désigne un nombre entier de motifs amide tel que le nombre de groupes ester représente de 10 % à 50 % du nombre total des groupes ester et amide ; chacun des symboles R₁ désigne indépendamment un groupe alkyle ou alcényle ayant au moins 4 atomes de carbone et notamment de 4 à 24 atomes de carbone ; chacun des symboles R₂ représente indépendamment un groupe hydrocarboné en C₄ à C₄₂ à condition que 50 % des groupes R₂ représentent un groupe hydrocarboné en C₃₀ à C₄₂ ; chacun des symboles R₃ représente indépendamment un groupe organique pourvu d'au moins 2 atomes de carbone, d'atomes d'hydrogène et optionnellement d'un ou plusieurs atomes d'oxygène ou d'azote ; et chacun des symboles R₄ représente indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure hétérocyclique définie par R₄-N-R₃, avec au moins 50 % des R₄ représentant un atome d'hydrogène.

12. Composition selon la revendication précédente, **caractérisée par le fait que** R₁ est un groupe alkyle en C₁₂ à C₂₂.

13. Composition selon l'une des revendications 11 ou 12, **caractérisée par le fait que** R₂ sont des groupes ayant de 30 à 42 atomes de carbone.

14. Composition selon l'une des revendications précédentes, **caractérisée par le fait que** le polymère est présent en une teneur allant de 0,01 % à 10 % en poids, par rapport au poids total de la composition, de préférence allant de 0,05 % à 5 % en poids, et mieux allant de 0,1 % à 3 % en poids.

15. Composition selon l'une des revendications précédentes, **caractérisée par le fait que** les particules de silice représentent de 0,1 à 12%, de préférence de 0,5 à 10, de préférence encore de 6 à 8% en poids par rapport au poids total de la composition.

16. Composition selon l'une quelconque des revendications précédentes, **caractérisée par le fait que** les particules réfléchissantes présentent une réflectance spectrale dans le spectre visible d'au moins 70 %.

17. Composition selon l'une quelconque des revendications précédentes, **caractérisée en ce que** les particules réfléchissantes présentent une dimension au plus égale à 250 µm, de préférence inférieure à 150 et mieux 100 µm.

18. Composition selon l'une quelconque des revendications précédentes, **caractérisée par le fait que** les particules réfléchissantes présentent une dimension au moins égale à 10 µm.

19. Composition selon la revendication 18, **caractérisée par le fait que** les particules réfléchissantes présentent une dimension allant de 20 à 80 µm.

20. Composition selon l'une quelconque des revendications précédentes, **caractérisée en ce que** les particules réfléchissantes sont présentes dans la composition à une teneur allant de 0,1 % à 20 %, en particulier de 1 à 15 %, notamment de 1 à 10 % par rapport au poids total de la composition.

21. Composition selon l'une quelconque des revendications précédentes, **caractérisée par le fait que** les particules réfléchissantes se présentent sous forme de plaquettes ou de sphères.

22. Composition selon l'une quelconque des revendications précédentes, **caractérisée par le fait que** les particules réfléchissantes comportent des particules comportant un substrat naturel ou synthétique enrobé au moins partiellement par une couche d'au moins un métal.

23. Composition selon la revendication 22, **caractérisée par le fait que** le métal est choisi parmi Ag, Au, Cu, Al, Zn, Ni, Mo, Cr et leurs mélanges ou alliages.

24. Composition selon la revendication 14, **caractérisée par le fait que** le métal est Ag ou un de ses alliages.

25. Composition selon l'une quelconque des revendications 22 à 24, **caractérisée par le fait que** le substrat est choisi parmi les substrats monomatière, multi-matériaux, les substrats organiques, les substrats inorganiques, les verres, les céramiques, les oxydes métalliques, les alumines, les silices, les silicates, notamment les aluminosilicates et les borosilicates, le mica synthétique et leurs mélanges.

26. Composition selon l'une quelconque des revendications 1 à 21, **caractérisée par le fait que** les particules réfléchissantes sont composées au moins en partie par des particules à substrat synthétique enrobé au moins partiellement d'au moins une couche d'au moins un composé métallique, notamment un oxyde métallique.

27. Composition selon la revendication 26, **caractérisée par le fait que** le substrat synthétique est choisi les substrats monomatière, multi-matériaux, les substrats organiques, les substrats inorganiques, les verres, les céramiques, les oxydes métalliques, les alumines, les silices, les silicates, notamment les aluminosilicates et les borosilicates, le mica synthétique, et leurs mélanges.

28. Composition selon l'une des revendications 26 ou 27, **caractérisée par le fait que** le composé métallique est choisi les oxydes de titane, notamment TiO₂, de fer, notamment Fe₂O₃, des oxydes d'étain, le sulfate de baryum et les composés MgF₂, CeF₃, ZnS, ZnSe, SiO₂, Al₂O₃, MgO, Y₂O₃, SeO₃, SiO, HfO₂, ZrO₂, CeO₂, Nb₂O₅, Ta₂O₅ et MoS₂ et leurs mélanges.

29. Composition selon la revendication 28, **caractérisée par le fait que** le composé métallique est un oxyde de titane, de fer ou d'étain ou un mélange de ceux-ci.

30. Composition selon la revendication 29, **caractérisée par le fait que** le composé métallique est TiO₂.

31. Composition selon l'une quelconque des revendications 1 à 30, **caractérisée par le fait que** les particules réfléchissantes sont composées au moins en partie par des particules formées d'un empilement d'au moins deux couches à indices de réfraction différents, notamment deux couches de polymères.

32. Composition selon l'une quelconque des revendications 1 à 31, **caractérisée par le fait que** les particules réfléchissantes sont composées au moins en partie par des particules d'au moins un oxyde métallique.

33. Composition selon la revendication 32, **caractérisée par le fait que** l'oxyde métallique est choisi parmi les oxydes de fer et de titane.

34. Composition selon l'une quelconque des revendications précédentes telles que les particules réfléchissantes sont présentes en une teneur allant de 0,01 % à 10 % en poids, par rapport au poids total de la composition.

35. Composition selon l'une quelconque des revendications précédentes telles qu'elle contient des pigments, des nacres et/ou des laques.

36. Composition selon l'une quelconque des revendications précédentes telles qu'elle comprend au moins un actif choisi parmi les hydratants, vitamines, acides gras essentiels, huiles essentielles, céramides, sphingolipides, filtres solaires liposolubles ou sous forme de nano-particules.

37. Composition selon l'une quelconque des revendications précédentes telles qu'elle comprend au moins un ingrédient choisi parmi les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

38. Composition selon l'une quelconque des revendications précédentes telles que le ratio massique entre le polymère et les particules de silice va de 1 :1000 à 1 :1, de préférence de 1 :100 à 1 :10, et de manière encore plus préférée de 5 :1000 à 5 :100.

39. Procédé de préparation d'une composition selon l'une des revendications précédentes **caractérisé en ce que** l'on mélange des particules de silice, des particules réfléchissantes et au moins un polymère de masse moléculaire moyenne en poids inférieure à 100 000, comportant a) un squelette polymérique ayant des motifs de répétition hydrocarbonés pourvus d'au moins un hétéroatome, et b) éventuellement au moins une chaîne grasse pendante et/ou au moins une chaîne grasse terminale éventuellement fonctionnalisées, ayant de 6 à 120 atomes de carbone et étant liées à ces motifs hydrocarbonés.

40. Gloss comprenant une composition cosmétique selon l'une des revendications 1 à 38.

41. Utilisation d'une composition cosmétique selon l'une des revendications 1 à 38 pour l'obtention d'un dépôt brillant.
